# EUROPÄISCHE PATENTSCHRIFT

(11) **EP 2 001 655 B1**
(45) Veröffentlichungstag und Bekanntmachung des Hinweises auf die Patenterteilung: **07.01.2015**
(21) Anmeldenummer: 07724068.7
(22) Anmeldetag: 05.04.2007
(51) Int. Cl.: B29C 65/16, A61M 39/10

(54) **DURCHSTRAHLLASERSCHWEISSVERFAHREN FÜR DIE VERBINDUNG VON KUNSTSTOFFFORMKÖRPERN**
TRANSMISSION LASER WELDING METHOD FOR JOINING MOLDED PLASTIC ARTICLES
PROCÉDÉ DE SOUDAGE LASER PAR TRANSMISSION DESTINÉ À LA CONNEXION DE CORPS MOULÉS EN PLASTIQUE

(30) Priorität: 06.04.2006 DE 102006016299
(43) Veröffentlichungstag der Anmeldung: 17.12.2008
(60) Teilanmeldung: 12151232.1
(73) Patentinhaber: Fresenius Medical Care Deutschland GmbH, 61352 Bad Homburg (DE)
(72) Erfinder: KREISCHER, Thomas, 66113 Saarbrücken (DE); KUGELMANN, Franz, 66606 St. Wendel/Bliesen (DE)
(74) Vertreter: Stolmár, Matthias
(86) Internationale Anmeldenummer: PCT/EP2007/003127
(87) Internationale Veröffentlichungsnummer: WO 2007/115803

(56) Entgegenhaltungen:
- EP-A- 0 087 403
- EP-A- 1 518 664
- EP-A- 1 609 555
- WO-A-2005/097471
- WO-A-2005/113049
- DE-A1-102005 010 193
- FR-A1- 2 597 379
- FR-A1- 2 812 372
- JP-A- 1 151 464
- US-A1- 2006 086 701

## Beschreibung

Die vorliegende Erfindung betrifft ein Durchstrahllaserschweißverfahren zum Verschweißen von insbesondere Schlauchsegmenten aus Kunststoff mit im Wesentlichen röhrenförmigen Formkörpern wie weitere Schlauchsegmente, Konnektoren, Fittings, Verschlüssen etc.

Zur formschlüssigen Verbindung von Formkörpern aus Kunststoffen, insbesondere aus verschiedenen Kunststoffen, existieren grundsätzlich verschiedene Möglichkeiten. Beispielsweise können die zwei Formkörper mittels Klebe- oder Schweißverfahren miteinander formschlüssig verbunden werden.

Unter den Schweißverfahren wird neben IR-Schweißen (siehe z.B. WO 2005/080067) das Schweißen mittels Laserstrahlung in letzter Zeit besonders intensiv untersucht.

Das grundlegende physikalische Prinzip eines Schweißverfahrens mittels Laser erfordert, daß mindestens ein Teil des eingesetzten Laserlichts von dem Material mindestens eines der beiden miteinander zu verbindenden Formteile in zumindest so einem Ausmaß absorbiert wird, daß es in Wärme umgewandelt werden kann und das Material an der durch das Laserlicht erwärmten Stelle durch Erwärmung fließfähig wird und eine schlüssige Verbindung mit dem Kunststoffmaterial des zweiten Formteils ermöglicht.

Das Schweißverfahren begrenzende Parameter sind die Wellenlänge des verwendeten Laserlichts und das Absorptionsverhalten des Kunststoffes bei dieser Wellenlänge. Insbesondere werden dabei Hochleistungsdiodenlaser mit einer Wellenlänge von 610-840 Nanometern und Nd:YAG-Festkörperlaser mit einer Wellenlänge von circa 1.050 Nanometern entweder im Bereich des sichtbaren Lichts (400-750 Nanometer) bzw. ein im infraroten Bereich verwendet. Es können allerdings auch oftmals CO₂-Gaslaser mit einer Wellenlänge von circa 11.000 Nanometern verwendet werden.

Die Absorptionseigenschaften und damit die Bearbeitung von Kunststoffen sind in Abhängigkeit von der verwendeten Wellenlänge der Laserstrahlung sehr unterschiedlich. Inhomogenitäten im Kunststoff, wie beispielsweise Pigmente, Füll- oder Verstärkungsstoffe, aber auch kristalline Überstrukturen bei teilkristallierenden Kunststoffen streuen die eingekoppelte Strahlung und setzen insbesondere die Eindringtiefe der Strahlung in den Kunststoff herab.

Trifft ein Laserstrahl auf ein zu erwärmendes Kunststoffteil, so wird die Laserstrahlung zu verschiedenen Teilen reflektiert, absorbiert und transmittiert. Die Intensitätsabnahme der in den Kunststoff eindringenden Strahlung lässt sich in Abhängigkeit von der Materialtiefe nach dem so genannten Bougourschen Gesetz beschreiben. Dabei nimmt die eingekoppelte Intensität exponentiell mit der Materialtiefe ab.

Probleme treten insbesondere durch die thermische Zersetzung durch Strahlungserwärmung von Kunststoffen, insbesondere mittels CO₂-Lasern auf. Dies liegt an der schlechten Wärmeleitfähigkeit von Kunststoffen, da die Oberflächentemperatur des Kunststoffteils oftmals schnell ansteigt, wobei die Gefahr einer thermischen Materialzersetzung besteht. [Die Grundlagen von Laserschweißverfahren sind beispielsweise bei H. Potente et al. "Laserschweißen von Thermoplasten" (Plastverarbeiter 1995, Nr. 9, S. 42 ff.), F. Becker et al. "Trends bei Serienschweißverfahren in Kunststoffe 87 (1997, S. 11 ff.) sowie von H. Puetz et al. in Modern Plastics, (1997, S. 121 ff.) dargestellt.]

Das Absorptionsverhalten und damit auch die Transmission von einem bei einer bestimmten Wellenlänge lasertransparenten Polymer bzw. Kunststoff kann z.B. durch das Zumischen von Absorbern gesteuert werden. Derartige Absorber sind beispielsweise Ruß bzw. auch spezielle Farbstoffe, die in den letzten Jahren entwickelt wurden.

Eine Reihe von Farbstoffen, die ein derartiges steuerbares Absorptionsverhalten ermöglichen, sind kommerziell erhältlich und wurden speziell dafür entwickelt, in Polymermischungen zugemischt zu werden, um eine Laserverschweißung bei definierten Wellenlängen zu ermöglichen. Dafür bieten sich auch die bei I.A. Jones et al. "Use of infrared dyes for Transmission Laser Welding of Plastics" (Tech 2000 Conference Proceedings, S. 1166 ff.) offenbarten Farbstoffe an.

Eine spezielle Form des Laserschweißverfahrens, nämlich Laserdurchstrahlschweißverfahren haben gegenüber anderen Schweißverfahren auch den Vorteil, daß auch komplexe Geometrien der Fügefläche schnell und rationell verschweißt werden können. Bei der Verschweißung von Schlauchstücken mit z. B. Konnektoren, Fittings etc. ist es nötig, daß ein Fügepartner vom Laserstrahl vollständig durchstrahlt wird, d. h. also Laserstrahlung nicht absorbiert. Der zweite bzw. Teile des zweiten Fügepartners müssen das Laserlicht unter Hitzeentwicklung absorbieren können. Üblicherweise erfolgt die Bestrahlung von der Außenseite eines Fügepartners aus.

Die EP 1552916 A1 beschreibt beispielsweise das Verbinden von röhrenförmigen Formkörpern mittels Laserschweißverfahren, wobei jedoch der Laser um die zu fügende Verbindung der Formkörper gedreht werden muss, was eine aufwendige Apparatur und Mechanik erfordert, damit zwei dreidimensionale Teile miteinander fugenlos verschweißt werden können.

Die US 2003/0141634 beschreibt laserverschweißbare Schläuche für medizinische Anwendungen, wobei in einem mehrschichtig aufgebauten Schlauch jede Schicht ein laserabsorbierendes Material umfasst. Auch hier ist es notwendig, daß entweder der Schlauch oder die gesamte Laserdurchstrahlapparatur einer Drehbewegung unterzogen werden muss, damit die Schläuche über ihren gesamten Umfang miteinander verschweißt werden können.

Die DE 10245355 A1 beschreibt die Verbindung eines Rohrelementes über eine Manschette. In einem so genannten Laserdurchstrahlverfahren werden ein Rohrabschnitt, ein so genannter Konnektor, und die Manschette miteinander verschweißt. Das Verschweißen erfordert hier ebenfalls eine um den gesamten Umfang durchgehende Schweißnaht, was nur durch das Drehen der Lasereinrichtung bzw. des zu verschweißenden Schlauches erfolgt.

Die WO 2005/063469 offenbart Schlauchstücke, die über sogenannte Manschetten miteinander verbunden werden können. Die Verbindung zwischen Schlauch und Manschette erfolgt über eine Verbindungsschicht, die ein bei 700 bis 2500 nm absorbierendes Material enthält. Hier erfordert der Laserschweißvorgang eine Rotationsbewegung entweder des Lasers bzw. des verschweißenden Schlauches.

Nachteilig bei diesem aus dem Stand der Technik bekannten Laserschweißverfahren ist es also insbesondere, daß die Fügeflächen zwischen den zwei miteinander zu verbindenden Formteilen ringsum vom Laser bestrahlt werden müssen. Dies setzt voraus, daß die miteinander zu verbindende Fläche dem Laser von allen Seiten zugänglich sein muss. Die Geometrie bestimmter Bauteile lässt eine umlaufende Bestrahlung oft nicht zu. Eine Rotation des entsprechenden Bauteils war oftmals geometriebedingt nicht durchführbar, so daß in vielen Fällen eine Laserverschweißung bislang unmöglich war.

Aufgabe der vorliegenden Erfindung war es daher, ein Laserdurchstrahlschweißverfahren bereitzustellen, das den oben erwähnten Nachteilen des Standes der Technik abhilft und insbesondere es ermöglicht, daß eine vollständige umlaufende Verschweißung zweier miteinander zu verbindender Formteile rotations- bzw. drehfrei ermöglicht wird. Ferner ist es Aufgabe der vorliegenden Erfindung eine Vorrichtung bereitzustellen, mit der zwei miteinander zu verbindende Formteile schnell verschweißt werden können, ohne ein Teil der Vorrichtung oder eines der zu verschweißenden Teile drehen zu müssen.

Erfindungsgemäß wird die Aufgabe durch eine Vorrichtung zum Verschweißen von zwei Fügepartnern bestehend aus einem Schlauchsegment und einem röhrenförmigen Formkörper gelöst, die einen Laser und einen Spiegel umfaßt, wobei der Laser, die Fügepartner und der Spiegel so angeordnet werden können, daß der Strahl des Lasers auf die Fügepartner und anschließend auf den Spiegel auftrifft, wobei der Spiegel teilweise die Kontur der inneren Fläche eines Hohlzylinders aufweist. Mit Hilfe dieser Vorrichtung kann die den Absorber enthaltende Schicht gleichmäßiger bestrahlt werden. Es kommt nicht zur Überhitzung punktueller Bereiche des Schlauches und die Schweißnaht wird einheitlicher.

In einer bevorzugten Ausführungsform ist der Strahl des Lasers in einem Strahlabschnitt divergent und der divergente Strahlabschnitt trifft zunächst auf das Schlauchsegment und anschließend auf den Spiegel. Vorteil dieser Ausführungsform ist eine noch gleichmäßigere Bestrahlung der den Absorber enthaltenden Schicht.

Üblicherweise werden die zu verbindenden Fügepartner, das Schlauchsegment und der röhrenförmige Formkörper mit einem Laserstrahl bestrahlt. Zum einen geht ein Großteil der bereitgestellten Laserenergie bei Verschweißen durch Streuungs- und Reflexionseffekte verloren. Alle nicht senkrecht auf die Schlauchoberfläche auftreffenden Strahlen werden abgelenkt, ab einem bestimmten Grenzwinkel werden die Strahlen totalreflektiert und tragen nicht zum Verschweißen der Fügepartner bei. Ferner ist die zu durchdringende laserabsorbierende Materialschicht in den Randbereichen wesentlich größer und trägt somit auch dazu bei, daß den unten liegenden Bereichen weniger Energie zugeführt wird. Durch die Reflexion der Strahlen an dem Spiegel wird Energie genutzt, die ansonsten durch Reflexion oder Transmission verloren gehen würde. Die am Spiegel reflektierten Strahlen können erneut in das Schlauchmaterial eindringen, um für ein Schweißverfahren zur Verfügung stehen.

Die Kontur des Spiegels beschreibt einen Teil einer inneren Oberfläche eines Hohlzylinders. Die Aussparung an einer Seite, die der vollkommen zylindrischen Kontur fehlt, dient als Blende für den einfallenden Laserstrahl. Leichte Abweichungen an der idealen Kontur eines Hohlzylinders sind möglich. Der Querschnitt des Hohlzylinders wäre dann nicht kreisrund, sondern elliptisch.

Ein Laserstrahl ist im Rahmen dieser Erfindung divergent, wenn der Strahlquerschnitt in Strahlrichtung größer wird. Der Vorteil dieser Anordnung liegt darin, daß bei Verwendung der Vorrichtung für ein Schweißverfahren alle Bereiche der den Absorber umfassenden Schicht eines Schlauchsegments gleichmäßig bestrahlt werden können. Dadurch werden Temperaturdifferenzen innerhalb der den Absorber umfassenden Schicht gering gehalten.

Bevorzugt besteht der Spiegel aus Aluminium oder einer Aluminiumlegierung. Es hat sich gezeigt, daß insbesondere Spiegel aus poliertem Aluminium die besten Reflexionseigenschaften aufweist. Spiegel aus Stahl erhitzen sich sehr stark und reflektieren unmerklich. Spiegel aus Messing erhitzen sich auch, reflektieren aber auch einen Teil der Strahlung. Der Reflexionseffekt geht mit zunehmender Abstumpfung der Spiegeloberfläche, die durch Erwärmung entsteht, zurück. Eine gut reflektierende Oberfläche kann aber durch erneutes Polieren wiederhergestellt werden.

Bevorzugt liegt der Durchmesser des Hohlzylinders zwischen 5 und 25 mm, noch bevorzugter bei 20 mm. Der Abstand zwischen Spiegeloberfläche und Oberfläche der Fügepartner sollte nicht zu groß sein, damit nicht zu viel am Spiegel reflektierte Lichtenergie abgestrahlt wird, ohne die Fügepartner zu durchdringen. Ferner können bevorzugt Schläuche mit einem Gesamtdurchmesser von bis zu 13 mm mit dieser Vorrichtung geschweißt werden. Schläuche mit größerem Durchmesser haben gewöhnlich größere Wandstärken. Insbesondere die keinen Absorber enthaltende Schicht ist dicker. Diese Schicht hat ein höheres Wärmeaufnahmevermögen. Mit dieser höheren Wärmeaufnahme wird dem Schweißprozeß Wärme entzogen, so daß es eher zu Schweißnahtfehlern kommen kann. Schlauchgröße und Abstand zwischen Spiegeloberfläche und Oberfläche der Fügepartner sind die begrenzenden Faktoren im Hinblick auf den Durchmesser des Hohlzylinders.

Bevorzugt wird bei der erfindungsgemäßen Vorrichtung ein Diodenlaser mit einer Wellenlänge von 750 bis 1000 nm, noch bevorzugter mit 808 nm, verwendet. Der Laser kann eine Leistung zwischen 380 und 520 W, bevorzugter eine Leistung von 500 W aufweisen. Mit den Diodenlaser können Laserstrahlen erzeugt werden, die konvergieren und divergieren können. Hierbei handelt es sich um sogenannte "aufgefächerte Diodenarray Laser". Ferner sind die Leistung des Lasers und die Wellenlänge des vom Laser emittierten Lichtes optimal für den Schweißprozeß, so daß keine Schweißnahtfehler entstehen und das polymere Material nicht überhitzt wird.

Die der Erfindung zugrundeliegende Aufgabe wird ferner dadurch gelöst, daß ein Laserschweißverfahren zur Verbindung eines PVC-freien Schlauchsegments mit einem röhrenförmigen Formkörper ("die Fügepartner") unter Verwendung eines Lasers und eines Spiegels bereitgestellt wird, wobei die Schlauchwand des Schlauchsegments aus mindestens zwei voneinander verschiedenen Materialschichten aufgebaut ist und eine dieser Schichten zumindest abschnittsweise einen Absorber für Laserstrahlung enthält, wobei bei dem Verfahren
a) das PVC-freie Schlauchsegment und der röhrenförmige Formkörper bereitgestellt werden,
b) das Schlauchsegment und der röhrenförmige Formkörper formschlüssig ineinandergeschoben werden, so daß sich die Oberflächenbereiche des Schlauchsegments und des röhrenförmigen Formkörpers überlappen,
c) das Schlauchsegment und der röhrenförmige Formkörper mit den sich überlappenden Oberflächenbereichen vor dem Spiegel angeordnet werden,
d) das Schlauchsegment und der röhrenförmige Formkörper mit den sich überlappenden Oberflächenbereichen mit Laserstrahlung bestrahlt werden, so daß die vom Schlauchsegment oder vom röhrenförmigen Formkörper reflektierten oder transmittierten Laserstrahlen von dem Spiegel reflektiert werden und teilweise wieder auf das Schlauchsegment oder den röhrenförmigen Formkörper auftreffen, wobei sich eine Schweißverbindung zwischen dem Schlauchsegment und dem röhrenförmigen Formkörper ausbildet.

Mit diesem Verfahren kann die Bestrahlungsdauer im Vergleich zu Schweißverfahren, bei denen zu verschweißende Fügepartner oder Teile der Vorrichtung bewegt werden müssen, erheblich verkürzt werden. Das durch Reflexion und Transmission an den Fügepartnern abgestrahlte Laserlicht wird am Spiegel reflektiert und steht für ein weiteres Schweißverfahren erneut zur Verfügung, so daß der Laser und/oder die zu verschweißenden Fügepartner nicht bewegt werden müssen. Daher verkürzt sich die Bestrahlungsdauer auf wenige Sekunden. Bevorzugt können die Fügepartner in weniger als 3 Sekunden miteinander verschweißt werden. Somit wird bei dem Verfahren in Schritt d) höchstens 3 Sekunden bestrahlt.

Um optimale Ergebnisse zu erreichen und eine einheitliche Schweißnaht zu bilden, wird bei dem Verfahren ein Spiegel verwendet, der die Kontur der inneren Fläche eines Hohlzylinders hat. Noch bevorzugter wird das Verfahren unter Verwendung der erfindungsgemäßen Vorrichtung durchgeführt.

Bei einer bevorzugten Ausführungsform dieses Verfahrens ist der Durchmesser des Laserstrahls beim Auftreffen auf den Schlauchabschnitt geringer als der Durchmesser des zu verschweißenden Schlauchabschnitts. Bevorzugt liegt das Verhältnis des Durchmessers des Laserstrahls zu dem Durchmesser des Schlauchabschnitts zwischen 1 zu 2 und 1 zu 5.

Bei dem erfindungsgemäßen Verfahren fällt jeglicher Verfahrweg weg, da mit Hilfe des Spiegels die Strahlung gleichmäßig auf die gesamte Fläche abstrahlt. Jeglicher Fahrweg fällt auch dann weg, wenn der Durchmesser des Strahlabschnitts beim Auftreffen auf den Schlauchabschnitt geringer ist als der Durchmesser des Schlauchabschnitts.

Ein weiterer Vorteil des erfindungsgemäßen Verfahrens besteht darin, daß sich ein gleichmäßigeres Schweißbild einstellt. Als positiver Nebeneffekt ist der gewonnene Platz zwischen Laseroptik und Schweißstelle zu sehen, wodurch die Fügepartner einfacher in die Halterung eingelegt und herausgenommen werden können.

Für den Fall, daß ein Spiegel eingesetzt wird, der die Kontur der inneren Fläche eines Hohlzylinders aufweist, ist der Abstand zwischen Spiegel und Schlauchwand ringsrum in etwa gleich. In dem Fall, daß der Spiegel der Vorrichtung nicht exakt die Kontur eines Hohlzylinders beschreibt, kann der Abstand zwischen Spiegel und Schlauchwand nicht gleich sein. Wenn der Querschnitt des Hohlzylinders elliptisch ist, sind die zu verbindenden Fügepartner zentriert vor dem Spiegel angeordnet.

Das erfindungsgemäße Verfahren bietet den Vorteil, daß die beiden Fügepartner nur von einer einzigen Seite aus durchstrahlt werden, wobei sich ringsum eine formschlüssige Schweißverbindung der Fügepartner ergibt. Dadurch kann das erfindungsgemäße Verfahren in Bezug auf entweder die Laservorrichtung oder die Fügepartner rotations- bzw. drehungsfrei durchgeführt werden, so daß eine weit weniger aufwendige mechanische Apparatur zur Durchführung des erfindungsgemäßen Verfahrens nötig ist als bei den aus dem Stand der Technik bekannten Verfahren, was somit eine wesentliche Vereinfachung darstellt.

Wird ein Verfahren ohne Verwendung eines Spiegels durchgeführt, kann es nötig sein, daß der Laser oder die Fügepartner eine Schiebestrecke durchfahren müssen, um gleichmäßige Schweißnähte zu erzeugen. Vorteil des Verfahrens unter Verwendung der erfindungsgemäßen Vorrichtung ist, daß eine gleichmäßigere Bestrahlung der Fügepartner möglich ist und keine Teile der Vorrichtung oder die zu verschweißenden Teile bewegt werden müssen.

Zur Durchführung des erfindungsgemäßen Verfahrens ist es dabei erfindungswesentlich, daß ein mehrschichtig aufgebautes Schlauchsegment bereitgestellt wird, wobei in einer Schicht ein Absorber für Laserstrahlung enthalten sein muß.

Der Begriff "formschlüssige" wird im Vorliegenden so verstanden, daß das Schlauchsegment und der rohrförmige Formkörper spalt- bzw. fugenfrei miteinander überlappen. Das Schlauchsegment übt dabei bevorzugt einen Anpreßdruck auf den rohrförmigen Formkörper aus.

Unter dem Begriff "Schlauchsegment" wird jeder beliebige Abschnitt eines Schlauches beliebiger Länge verstanden, durch den Flüssigkeiten durchgeleitet werden können. Da ein Einsatz als medizinischer Schlauch beispielsweise in Hämodialysesystemen vorgesehen sein kann, ist es nötig, daß der Schlauch insbesondere PVC-frei ausgestaltet ist, da neuere Studien ergeben haben, daß die in PVC (Polyvinylchlorid) verwendeten Weichmacher durch die durch den Schlauch hindurchgeführten biologischen Flüssigkeiten gelöst werden und gesundheitliche Gefahren in einem menschlichen Organismus hervorrufen können.

Die Verschweißbarkeit bzw. die Stabilität der Schweißverbindung wird insbesondere durch die Menge an Absorber für das verwendete Laserlicht in der den Absorber enthaltenden Schicht bestimmt. Erfindungsgemäß enthält eine äußere Oberfläche des Schlauches, d.h. entweder dessen Innenseite oder dessen Außenseite eine funktionalisierte, d.h. den Absorber enthaltende dünne Schicht. Diese Schicht wird mit der darunterliegenden zweiten Schicht typischerweise koextrudiert. Die Schichtdicken der den Absorber enthaltenden Schicht liegen im Bereich von 20 bis 100 µm, so daß sie auch noch im unteren Schichtdickenbereich durch Koextrusion herstellbar sind.

Die Anordnung der den Absorber enthaltenden Schicht entweder als die das Schlauchinnere oder in einer anderen Ausführungsform der Erfindung als die das Schlauchäußere bildenden Oberfläche ermöglicht insbesondere verschiedene geometrische Anordnungen von miteinander zu verschweißenden Formteilen: Zum Beispiel kann in einer bevorzugten Ausführungsform der Erfindung der Schlauch in den röhrenförmigen Formkörper, beispielsweise also in einen Konnektor, gesteckt werden, so daß in diesem Fall die äußere Schicht mit dem Absorber funktionalisiert sein muss, d.h. die Schicht, die mit dem zu verschweißenden zweiten Fügeteil, dem Konnektor, in Berührung kommt.

In einer weiteren vorteilhaften Ausführungsform der Erfindung bildet die funktionalisierte Schicht, d.h. die Schicht, die den Absorber enthält, die Innenseite des Schlauchsegmentes, so daß ein röhrenförmiger Formkörper in den Schlauch eingeführt werden kann.

In weiteren bevorzugten Weiterbildungen der Erfindung enthält nur der Teil der Schicht den Absorber, der mit dem zu verschweißenden Bereich des röhrenförmigen Formkörpers überlappt. Dies ist selbstverständlich bei beiden vorstehend beschriebenen geometrischen Alternativen möglich. Damit kann insbesondere auch der Einsatz von teuren Farbstoffen für die Absorption ermöglicht werden.

Die Konzentration des Absorbers in der ihn enthaltenden Schicht beträgt typischerweise zwischen 50 bis 300 ppm, bevorzugt 80-200 ppm, und wird so gewählt, daß nur ca. 10-50%, bevorzugt 15-40% des eingesetzten Laserlichts entlang der Durchtrittsweglänge des Laserlichts durch die den Absorber enthaltenden Schicht des ersten im Wesentlichen halbzylindrischen Segments absorbiert werden und somit für die Verschweißung des ersten Teils verwendet werden können. In diesem Zusammenhang versteht es sich, daß der Fachmann Art und Menge des Absorbers auf die Art des eingesetzten Lasers bzw. dessen Wellenlänge abstimmt, um die vorgenannten Werte zu erzielen.

Vorteilhafterweise bestehen die Schichten des erfindungsgemäß einsetzbaren Schlauchsegments aus PVC-freien Materialien, insbesondere besteht die den Absorber enthaltende Schicht aus Polyolefinen, wie beispielsweise Polyethylen, Polypropylen, Polyisopren, außerdem Styrol-Olefin Copolymere, wie z. B. Styrol-Ethylen-Butadien-Styrol Blockcopolymere (SEBS), Styrol-Ethylen-Propylen-Styrol Blockcopolymere (SEPS), Styrol-Isopren-Styrol Copolymere (SIS), Styrol-Ethylen-Butadien Copolymere (SEB) sowie Mischungen davon.

Die mindestens eine weitere absorberfreie Schicht nimmt insbesondere die Funktion ein; dem Schlauchsegment eine gewisse mechanische Stabilität, Knickresistenz, etc. zu verleihen.

Dafür sind als Materialien für diese Schicht insbesondere reine polyolefinische Materialien wie Polypropylen, Polyethylen, Polyisopren und Mischungen davon besonders bevorzugt. In weiteren bevorzugten Ausführungsformen können selbstverständlich auch Styrol-Ethylen-Butadien-Styrol Blockcopolymere (SEBS), Styrol-Ethylen-Propylen-Styrol Blockcopolymere (SEPS), Styrol-Isopren-Styrol Copolymere (SIS), Styrol-Ethylen-Butadien Copolymere (SEB) sowie Mischungen davon bzw. mit den reinen Polyolefinen verwendet werden.

In weiteren Ausführungsformen der Erfindung hat das erfindungsgemäß verwendete Schlauchsegment einen inneren Durchmesser von maximal 8 mm und einen äußeren maximalen Durchmesser von 12 mm. Bevorzugte Wandstärken des Schlauchsegments liegen bei 2 mm, in weiteren spezifischen Ausführungsformen wie z. B. bei einer vorgesehenen Verwendung des Schlauches als so genannter Blutsystemschlauch sogar bei bis zu 1,5 mm.
In weiteren vorteilhaften Ausführungsformen kann der Schlauch noch weitere beliebige Abfolgen von Schichten enthalten. Wichtig ist nur, daß diese weiteren Schichten das Laserschweißverfahren nicht beeinträchtigen, so daß diese Schichten im Wesentlichen lasertransparent sein müssen.

Damit können auch komplexe Anforderungen insbesondere hinsichtlich mechanischer Stabilität, Knickresistenz, Elastizität, etc. ausreichend berücksichtigt werden.

Bevorzugt wird im Rahmen der erfindungsgemäßen Verfahren ein so genannter Diodenlaser eingesetzt. Dieser weist eine Wellenlänge bis zu 1000 nm, bevorzugt von 750 bis 900 nm, am meisten bevorzugt von 808 nm auf. Besonders wichtig ist bei der Durchführung der erfindungsgemäßen Verfahren, daß mit oder ohne Anwendung von Druck eine Fügeebene erhalten wird, bei der beide Fügepartner bevorzugt fugenlos miteinander überlappen, so daß die formschlüssige Verbindung durch das durch den Laser erhitzte fließfähige Polymer besonders einfach erhalten wird, so daß eine große Vielfalt bei der Gestaltung der Schweißnahtgeometrie erfindungsgemäß erhalten werden kann. Die am häufigsten verwendete Geometrie beim Fügen von Kunststoffen mit einem Laser, insbesondere mit einem Diodenlaser, ist die Überlappnaht, die auch im vorliegenden Fall bevorzugt eingesetzt wird.

In diesem Zusammenhang ist wichtig, daß die Vielzahl möglicher Lasertypen, die im Rahmen der vorliegenden Erfindung zum Einsatz vorgesehen sind (z. B. Nd:YAG Laser für den Fall, daß das Verfahren ohne Verwendung eines Spiegels durchgeführt wird, oder ein Diodenlaser für die erfindungsgemäßen Verfahren, ob mit oder ohne Verwendung eines Spiegels, usw.), die dadurch auch unterschiedliche Wellenlängen aufweisen, aufgrund der großen Energiedichte der Laserstrahlung keine Einschränkung hinsichtlich der Absorbersubstanzen bedeuten. Durch die große Energiedichte ist es nicht notwendig, im Absorptionsmaximum der jeweiligen gewählten Absorbersubstanz einzustrahlen. Es genügt, wenn in einem Fenster von circa ± 100 nm um das Maximum der jeweiligen Substanz herum eingestrahlt wird, so daß eine breite Palette von Absorbern für den Einsatz in dem erfindungsgemäßen Verfahren Verwendung findet. Gewöhnlich wird bei dem Verfahren, das die Schritte a) bis e) umfaßt, ein Laser eingesetzt, der weitestgehend parallele Lichtstrahlen emittiert.

In dem erfindungsgemäßen Verfahren, bei dem kein Spiegel für die Reflexion der Laserstrahlen zum Einsatz kommt, ist es besonders bevorzugt, daß die Abschwächung der Laserstrahlung entlang der Durchtrittsweglänge des Laserlichts durch die den Absorber enthaltenden Schicht des ersten im Wesentlichen halbzylindrischen Segments aufgrund des Absorbers ca. 10-50%, bevorzugt 15-40% beträgt. Diese Bereiche ermöglichen, daß auch bei längeren Durchtrittswegen an einzelnen Kreissegmenten des Schlauchs noch genug Laserstrahlung das entgegengesetzte Kreissegment des Pumpschlauchs erreicht, so daß auch dort noch eine feste Verschweißung ermöglicht wird.

Für den Fall, daß das Verfahren ohne Verwendung eines Spiegels eingesetzt wird, beträgt die Laserleistung bei einem Diodenlaser üblicherweise ca. 400 Watt. Es versteht sich, daß diese Werte nur Anhaltspunkte für den Fachmann bei der Wahl des geeigneten Lasers sind. Auch bei diesem Verfahren kann als Laser ein "aufgefächerter Diodenarray Laser" mit einer Leistung von circa 400 Watt verwendet werden, der Strahlung auf einen Spot von circa 10 mm Durchmesser fokussiert. Typische Zeiten für die Einwirkung der Laserstrahlung betragen 1-10 s, bevorzugt 2-8 s, ganz besonders bevorzugt 3-7 s, je nach Schlauchdicke. Beispielhafte Zeiten betragen im Falle eines Blutpumpschlauchs mit einem Durchmesser von circa 12 mm 7 Sekunden, bei einem Schlauch mit einem Durchmesser von 6,5 mm circa 3 Sekunden bis zur Ausbildung einer festen Schweißverbindung.

Die Aufgabe der vorliegenden Erfindung wird ferner gelöst durch eine lasergeschweißte Fügeverbindung zwischen einem Schlauchsegment und einem röhrenförmigen Formkörper, die durch die erfindungsgemäßen Verfahren, ob unter Verwendung mit oder ohne Spiegel, hergestellt werden. Damit sind lasergeschweißte Fügeverbindungen, die ohne besonders großen mechanischen Aufwand hergestellt werden können, insbesondere im medizinischen Bereich einsetzbar, hier insbesondere bei der Förderung von physiologischen Flüssigkeiten, Infusionslösungen oder Blut.

An die zu verschweißenden Schlauch- und Anschlussstücke werden dabei besondere Anforderungen gestellt, so daß die Wahl der entsprechenden Polymere in Bezug auf Transparenz im sichtbaren Wellenlängenbereich, Hitzesterilisierbarkeit und Biokompatibilität beachtet werden müssen, die jedoch aus dem vorstehend aufgeführten Stand der Technik bekannt sind. Daher umfasst die vorliegende Erfindung ebenfalls ein Schlauchsystem, das eine Mehrzahl von derartigen lasergeschweißten Fügeverbindungen zwischen einem Schlauchsegment und einem röhrenförmigen Formkörper, beispielsweise einem Konnektor oder einem weiteren Schlauchsegment umfasst, zur Verwendung beispielsweise in einem Hämodialysekreislauf.

Die Erfindung ist weiter anhand von Ausführungsbeispielen in den nachstehenden Figuren näher erläutert, ohne daß dies als Einschränkung des Erfindungsgedankens verstanden werden soll.

Es zeigen:
- Fig. 1: einen Querschnitt durch eine Verbindung, bei der ein Konnektor auf ein Schlauchsegment geschoben wurde.
- Fig. 2: zeigt eine Verbindung, bei der ein Konnektor in einen Schlauch geschoben wurde.
- Fig. 3: zeigt eine lasergeschweißte Fügeverbindung zwischen einem Konnektor und einen in den Konnektor eingeschobenen Schlauch.
- Fig. 4: zeigt eine Anordnung bestehend aus einem Laser und einem hohlzylindrischen Spiegel.

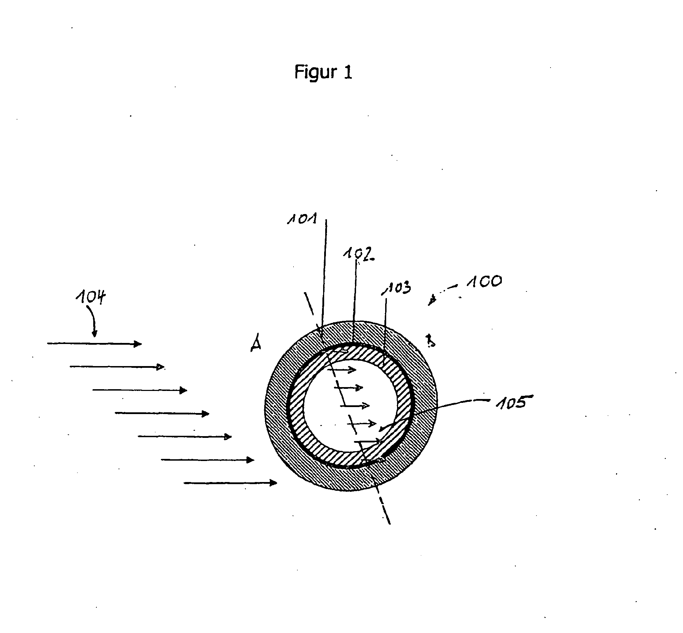

Figur 1 zeigt eine erfindungsgemäße Kombination 100 zwischen einem Konnektor 101 aus Polypropylen und einem Schlauch, der aus einer inneren Schicht 103 sowie aus einer äußeren Schicht 102 besteht, wobei die äußere Schicht 102 ein laserabsorbierendes Material wie beispielsweise einen Farbstoff enthält. Die Schicht 102 weist dabei eine Dicke von ca. 50 µm auf. Als Absorbersubstanz kann beispielsweise Lumogen IR 788 von der Firma BASF verwendet werden, jedoch ist es im Rahmen der vorliegenden Erfindung ebenfalls möglich, daß jeder andere beliebige Absorber, der im Wellenlängenbereich des verwendeten Diodenlasers absorbiert, verwendet wird. Nachdem das Schlauchsegment in den Konnektor 101 geschoben ist, wird eine Laserstrahlung 104 mit einer Wellenlänge von 808 nm über einen Zeitraum von 3-5 Sekunden angelegt. Die Laserstrahlung 104 wird in einem halbzylinderförmigen Bereich, der mit dem Großbuchstaben "A" dargestellt wird, von dem Absorber in der Schicht 102 absorbiert, und schmilzt den Kunststoff auf, so daß eine formschlüssige Verbindung zwischen dem Konnektor 101 aus Polypropylen und dem Schlauch an der äußeren Schicht 102 entsteht. Die Strahlung 104 wird absorbiert und trifft , wie vorstehend detailliert erläutert, um circa 10-50% abgeschwächt, dargestellt durch die Pfeile 105, in das zweite halbkreisförmige Segment B auf, wo sie ebenfalls von dem Absorber in der Schicht 102 absorbiert wird und somit auch im Segment B eine formschlüssige Verbindung durch Aufschmelzen der Schicht 102 zwischen der Schicht 102 des Schlauchsegmentes und dem Konnektor 101 aus Polypropylen hervorruft. Die Reststrahlung wird üblicherweise nicht völlig absorbiert. Aus den Figuren ist auch die zu durchlaufende unterschiedliche Weglänge für die Wellenfront des Laserlichts erkennbar: die Weglänge l₁ ist dabei viel kleiner als die Weglänge l₂, so daß Licht entlang der Weglänge l₁ weniger abgeschwächt wird, also eher im Bereich von 10% Abschwächung liegt, als entlang der Weglänge l₂, wo die Absorption eher bei 50% liegt.

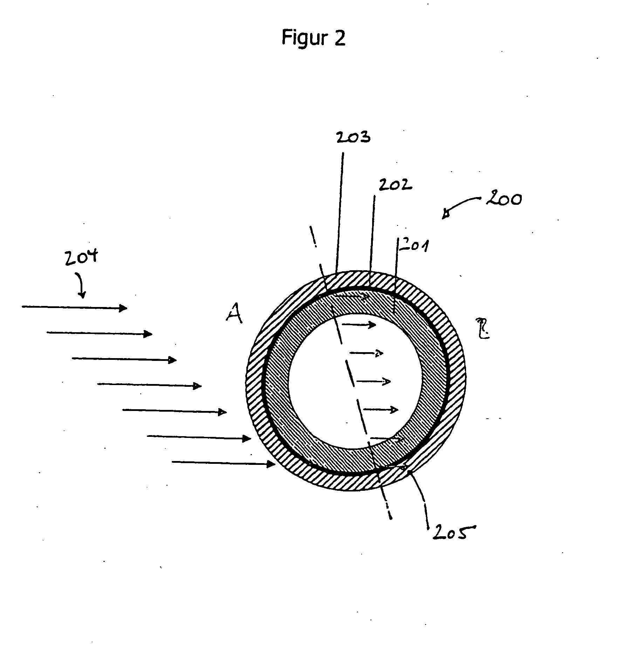

Figur 2 zeigt eine weitere Ausführungsform einer Verbindung 200, die im Rahmen des erfindungsgemäßen Verfahrens eingesetzt werden kann. Dabei wird ein Konnektor 201, beispielsweise aus Polypropylen in ein Schlauchsegment eingeführt, so daß die innenliegende Oberfläche des Schlauchs 202 das laserabsorbierende Material enthält und die nach außen liegende Schicht 203 zur Stabilisierung dient.

Es versteht sich, daß natürlich noch weitere Schichten außen bzw. zwischen den Schichten 202 und 200 angeordnet sein können, solange diese, wie vorstehend schon erwähnt, lasertransparent sind. Die Dicke der Schicht 202 beträgt ebenfalls 50 µm und als absorbierende Substanz wurde ebenfalls Lumogen IR 788 von BASF verwendet.

Die Anordnung 200 wird nun ebenfalls mit Laserstrahlung einer Wellenlänge λ = 808 nm bestrahlt (dargestellt mit Pfeil 204), die im ersten Halbkreis bzw. halbzylinderförmigen Segment A zu ca. 10-50 % absorbiert wird, so daß eine formschlüssige Verbindung zwischen der Oberfläche des Konnektors 201 und der dieStrahlung absorbierenden Schicht 202 entsteht. Die nicht absorbierte Strahlung 205 trifft auf die Schicht 202 im halbzylinderförmigen Segment B 202 auf und führt dort ebenfalls zur Absorption, Erhitzung und Aufschmelzung, so daß eine vollständige Verschweißung im gesamten Umfang des Schlauchsegments mit dem Konnektor erzielt wird.

Eine Rotation des Lasers bzw. der zu verbindenden Fügeelemente 100 bis 200 ist, wie hier gezeigt wurde, mit dem erfindungsgemäßen Verfahren zur Ausbildung einer umlaufenden Schweißnaht somit nicht erforderlich.

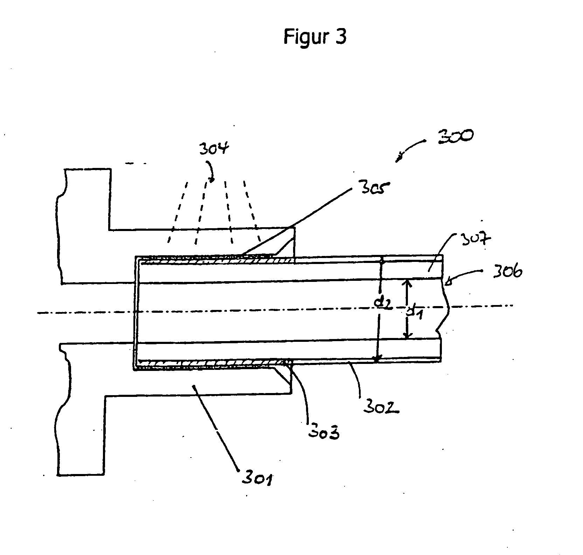

Figur 3 zeigt eine vollständig verschweißte Fügeverbindung 300 zwischen einem Konnektor 301 aus Polypropylen und einem Schlauchsegment 306, das aus einer inneren Schicht 307, die frei von laserabsorbierenden Materialien ist, sowie einer äußeren Schicht 302 die bereichsweise im Abschnitt 303, der mit der Innenseite des Konnektors 301 überlappt, einen Absorber für Laserlicht enthält. Nach Einwirken der Laserstrahlung 304 entsteht, wie in den Figuren 1 und 2 beschrieben, eine formschlüssige umlaufende Schweißnaht 305.

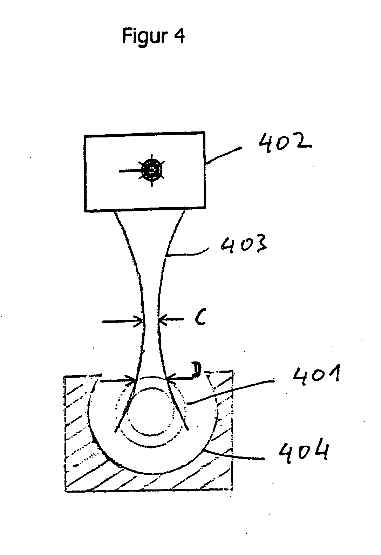

Figur 4 zeigt eine Vorrichtung 400 zum Verschweißen eines Schlauchsegments 401 bestehend aus einem Laser 402 und einem Spiegel 404. Der Diodenarray Laser emittiert einen Lichtstrahl 403, der in einem ersten Abschnitt konvergiert, in der Ebene C den geringsten Querschnitt aufweist und anschließend divergiert. Der divergente Strahlabschnitt trifft in der Ebene D auf das Schlauchsegment 401 auf. Gestreute und hindurchtretende Laserstrahlen treffen anschließend auf den Spiegel 404 auf, werden dort reflektiert und treffen teilweise wieder auf das Schlauchsegment auf. Durch diese Anordnung wird eine gleichmäßige Bestrahlung der den Absorber umfassenden Schicht gewährleistet, so daß das Schlauchsegment gleichmäßig verschweißt wird.

### Ausführungsbeispiel 1

In einer bevorzugten Ausführung wurde ein PVC-freier Schlauch mit folgendem Aufbau verwendet:

| | |
|---|---|
| Innendurchmesser | 8 mm |
| Außendurchmesser | 12 mm |
| Wandstärke: | 2 mm |

Die den Absorber enthaltende Schicht des Schlauches weist eine Zusammensetzung wie folgt auf:

| | |
|---|---|
| Dicke der Absorberschicht: | 50 µm |
| Zusammensetzung: | 60 % SEBS Tuftec 1221 (Asahi) |
| | 40 % PP-R RD204 CF (Borealis) |
| | 100 ppm Lumogen IR 788 BASF. |

Die absorberfreie Schicht bestand aus einer Mischung aus 70% SEBS Tuftec 1221 (Asahi) und 30% SIS (Hybrar 7125 F, Kuraray).

Das Schlauchsegment mit den beiden Schichten wurde durch Koextrusion hergestellt.

Als zweiter Fügepartner diente ein sogenannter Konnektor aus Polypropylen. Im Ausführungsbeispiel wird das Schlauchsegment wie in Figur 3 beschrieben in den Konnektor aus Polypropylen geschoben, mit dem Unterschied daß die gesamte äußere Schicht den Absorber enthielt.

Die ineinandergesteckten Fügepartner, das Schlauchsegment und der Konnektor wurden mit einem Diodenlaser der Firma Laserline, D-Mühlheim (Diodenlaser LDF 1000-500) mit einer Wellenlänge von 808 nm von einer einzigen Seite aus erfindungsgemäß bestrahlt. Es wurde eine so genannte Durchstrahlschweißung mit Überlappung im Rahmen des erfindungsgemäßen Verfahrens eingesetzt. Eine formschlüssig umlaufende Schweißnaht wurde so erfindungsgemäß ohne die Drehung eines oder beider Fügepartner erhalten.

Die so erhaltene Schweißverbindung erfüllt alle Anforderungen an Dichtigkeit, Zugfestigkeit und Hitzesterilisierbarkeit wie sie im medizinischen Bereich auftreten. Die Zugfestigkeit ("Schlauchausreißfestigkeit") wurde nach DIN 53455 bestimmt und betrug circa 170 N. Im vorliegenden Fall zerriss der Schlauch, während sich die geschweißte Verbindung auch bei dieser Krafteinwirkung nicht löste. Typischerweise betrug die Untergrenze der Zugfestigkeit erfindungemäß verschweißter Verbindungen 120 N und je nach verschweißter Fläche max. 160-170 N, wobei - wie vorstehend ausgeführt - eine genaue Messung der Obergrenze durch das Reißen des jeweiligen Schlauches nicht exakt bestimmt werden konnte.

## Patentansprüche

1. Laserschweißverfahren zur Verbindung eines PVC-freien Schlauchsegments mit einem röhrenförmigen Formkörper unter Verwendung eines Lasers und eines Spiegels, wobei die Schlauchwand des Schlauchsegments aus mindestens zwei voneinander verschiedenen Materialschichten aufgebaut ist und eine dieser Schichten zumindest abschnittsweise einen Absorber für Laserstrahlung enthält, wobei bei dem Verfahren
a) das PVC-freie Schlauchsegment und der röhrenförmige Formkörper bereitgestellt werden,
b) das Schlauchsegment und der röhrenförmige Formkörper formschlüssig ineinandergeschoben werden, so daß sich die Oberflächenbereiche des Schlauchsegments und des röhrenförmigen Formkörpers überlappen,
c) das Schlauchsegment und der röhrenförmige Formkörper mit den sich überlappenden Oberflächenbereichen vor dem Spiegel angeordnet werden,
d) das Schlauchsegment und der röhrenförmige Formkörper mit den sich überlappenden Oberflächenbereichen mit Laserstrahlung bestrahlt werden, so daß die vom Schlauchsegment oder vom röhrenförmigen Formkörper reflektierten oder transmittierten Laserstrahlen von dem Spiegel reflektiert werden und teilweise wieder auf das Schlauchsegment oder den röhrenförmigen Formkörper auftreffen, wobei sich eine Schweißverbindung zwischen dem Schlauchsegment und dem röhrenförmigen Formkörper ausbildet.

2. Verfahren nach Anspruch 1, bei dem der Spiegel die Kontur der inneren Fläche eines Hohlzylinders hat.

3. Verfahren nach einem der Ansprüche 1 oder 2, **dadurch gekennzeichnet, daß** der Absorber in der die innere Oberfläche des Schlauchsegments bildenden Schicht enthalten ist.

4. Verfahren nach Anspruch 3, **dadurch gekennzeichnet, daß** in Schritt b) der röhrenförmige Formkörper in das Schlauchsegment geschoben wird.

5. Verfahren nach einem der Ansprüche 1 oder 2, **dadurch gekennzeichnet, daß** der Absorber, in der die äußere Oberfläche des Schlauchsegments bildenden Schicht enthalten ist.

6. Verfahren nach Anspruch 5, **dadurch gekennzeichnet, daß** in Schritt b) das Schlauchsegment in den röhrenförmigen Formkörper geschoben wird.

7. Verfahren nach einem der vorhergehenden Ansprüche, **dadurch gekennzeichnet, daß** der Absorber in einer Konzentration von 50-300 ppm in der ihn enthaltenden Schicht enthalten ist.

8. Verfahren nach Anspruch 7, **dadurch gekennzeichnet, daß** die den Absorber enthaltende Schicht aus einem PVC-freien Material besteht.

9. Verfahren nach Anspruch 8, **dadurch gekennzeichnet, daß** das Material ausgewählt ist aus Polyethylen, Polypropylen, Polyisopren, Styrol-Olefin Blockcopolymere wie SEBS, SEPS, SIS, SEB und Mischungen davon.

10. Verfahren nach einem der Ansprüche 7, 8 oder 9, **dadurch gekennzeichnet, daß** die Schichtdicke der den Absorber enthaltenden Schicht 20 bis 100 µm beträgt.

11. Verfahren nach einem der Ansprüche 1 bis 6, **dadurch gekennzeichnet, daß** das Material der absorberfreien Schicht ausgewählt ist aus Polyethylen, Polypropylen, Polyisopren, Styrol-Ethylen-Butadien-Styrol Blockcopolymere (SEBS), Styrol-Ethylen-Propylen-Styrol Blockcopolymere (SEPS), Styrol-Isopren-Styrol Copolymere (SIS), Styrol-Ethylen-Butadien Copolymere (SEB) und Mischungen davon.

12. Verfahren nach einem der vorhergehenden Ansprüche, **dadurch gekennzeichnet, daß** das Schlauchsegment eine Wandstärke von maximal 4 mm aufweist.

13. Verfahren nach einem der vorhergehenden Ansprüche, **dadurch gekennzeichnet, daß** der röhrenförmige Formkörper ein Konnektor oder ein weiteres Schlauchsegment ist.

14. Verfahren nach Anspruch 13, **dadurch gekennzeichnet, daß** der Konnektor im wesentlichen aus Polypropylen besteht.

15. Verfahren nach einem der vorhergehenden Ansprüche, **dadurch gekennzeichnet, daß** das Schlauchsegment weitere Schichten umfasst.

16. Verfahren nach Anspruch 15, **dadurch gekennzeichnet, daß** die weiteren Schichten für Laserstrahlung durchlässig sind.

17. Lasergeschweißte Fügeverbindung zwischen einem Schlauchsegment und einem röhrenförmigen Formkörper, hergestellt durch das Verfahren gemäß einem der vorhergehenden Ansprüche.

18. Schlauchsystem enthaltend eine Mehrzahl von lasergeschweißten Fügeverbindungen zwischen einem Schlauchsegment und einem röhrenförmigen Formkörper nach Anspruch 17, zur Verwendung in einem Hämodialysekreislauf.

19. Vorrichtung zum Verschweißen von zwei Fügepartnern bestehend aus einem Schlauchsegment und einem röhrenförmigen Formkörper, die einen Laser und einen Spiegel umfaßt, wobei der Laser, die Fügepartner und der Spiegel so anordbar sind, daß der Strahl des Lasers auf die Fügepartner und anschließend auf den Spiegel auftrifft, **dadurch gekennzeichnet, daß** der Spiegel teilweise die Kontur der inneren Fläche eines Hohlzylinders aufweist.

20. Vorrichtung nach Anspruch 19, **dadurch gekennzeichnet, daß** der Strahl des Lasers in einem Strahlabschnitt divergiert und der divergente Strahlabschnitt auf die Fügepartner auftrifft.

21. Vorrichtung nach Anspruch 19 oder Anspruch 20, **dadurch gekennzeichnet, daß** der Spiegel aus Aluminium oder einer Aluminiumlegierung besteht.

22. Vorrichtung nach einem der Ansprüche 19 bis 21, **dadurch gekennzeichnet, daß** der Durchmesser des Hohlzylinders zwischen 5 und 25 mm liegt.

23. Vorrichtung nach Anspruch 22, **dadurch gekennzeichnet, daß** der Durchmesser des Hohlzylinders 20 mm beträgt.

24. Vorrichtung nach einem der Ansprüche 19 bis 23, **dadurch gekennzeichnet, daß** der Laser ein Diodenlaser ist, der Licht einer Wellenlänge von 750 bis 1000 nm emittiert.

25. Vorrichtung nach Anspruch 24, **dadurch gekennzeichnet, daß** der Laser Licht einer Wellenlänge von 808 nm emittiert.

26. Vorrichtung nach Anspruch 24 oder Anspruch 25, **dadurch gekennzeichnet, daß** der Laser eine Leistung von 380 bis 520 W hat.

27. Vorrichtung nach Anspruch 26, **dadurch gekennzeichnet, daß** der Laser eine Leistung von 500 W hat.

## Claims

1. A laser welding method for connecting a PVC-free tube segment to a tubular molded article using a laser and a mirror, wherein the tube wall of the tube segment is constructed from at least two material layers that differ from one another, and one of these layers contains, at least in sections, an absorber for laser radiation, wherein in the method
a) the PVC-free tube segment and the tubular molded article are provided,
b) the tube segment and the tubular molded article are inserted into one another in a positive-fit manner in such a way that the surface regions of the tube segment and of the tubular molded article overlap,
c) the tube segment and the tubular molded article are arranged, with the overlapping surface regions, in front of the mirror,
d) the tube segment and the tubular molded article, with the overlapping surface regions, are irradiated with laser radiation in such a way that the laser beams reflected or transmitted from the tube segment or from the tubular molded article are reflected by the mirror and partially strike the tube segment or the tubular molded article again, thereby forming a weld connection between the tube segment and the tubular molded article.

2. The method according to claim 1, in which the mirror has the contour of the inner surface of a hollow cylinder.

3. The method according to any one of claims 1 or 2, **characterized in that** the absorber is contained in the layer which forms the inner surface of the tube segment.

4. The method according to claim 3, **characterized in that** in step b) the tubular molded article is inserted into the tube segment.

5. The method according to any one of claims 1 or 2, **characterized in that** the absorber is contained in the layer which forms the outer surface of the tube segment.

6. The method according to claim 5, **characterized in that** in step b) the tube segment is inserted into the tubular molded article.

7. The method according to any one of the preceding claims, **characterized in that** the absorber is contained in the absorber-containing layer in a concentration of 50-300 ppm.

8. The method according to claim 7, **characterized in that** the absorber-containing layer is composed of a PVC-free material.

9. The method according to claim 8, **characterized in that** the material is selected from polyethylene, polypropylene, polyisoprene, styrene-olefin block copolymers such as SEBS, SEPS, SIS, SEB, and mixtures thereof.

10. The method according to any one of claims 7, 8 or 9, **characterized in that** the layer thickness of the layer containing the absorber is 20 to 100 µm.

11. The method according to any one of claims 1 to 6, **characterized in that** the material of the absorber-free layer is selected from polyethylene, polypropylene, polyisoprene, styrene-ethylene-butadiene-styrene block copolymers (SEBS), styrene-ethylene-propylene-styrene block copolymers (SEPS), styrene-isoprene-styrene copolymers (SIS), styrene-ethylene-butadiene copolymers (SEB) and mixtures thereof.

12. The method according to any one of the preceding claims, **characterized in that** the tube segment has a maximum wall thickness of 4 mm.

13. The method according to any one of the preceding claims, **characterized in that** the tubular molded article is a connector or a further tube segment.

14. The method according to claim 13, **characterized in that** the connector is composed substantially of polypropylene.

15. The method according to any one of the preceding claims, **characterized in that** the tube segment comprises further layers.

16. The method according to claim 15, **characterized in that** the further layers are permeable to laser radiation.

17. A laser-welded joining connection between a tube segment and a tubular molded article, produced by the method according to any one of the preceding claims.

18. A tube system, containing a plurality of laser-welded joining connections between a tube segment and a tubular molded article according to claim 17, for use in a hemodialysis circuit.

19. A device for welding two joining partners composed of a tube segment and a tubular molded article, the device comprising a laser and a mirror, wherein the laser, the joining partners and the mirror can be arranged in such a way that the beam of the laser strikes the joining partners and subsequently strikes the mirror, **characterized in that** the mirror partially has the contour of the inner surface of a hollow cylinder.

20. The device according to claim 19, **characterized in that** the beam of the laser diverges in a beam portion, and the divergent beam portion strikes the joining partners.

21. The device according to claim 19 or claim 20, **characterized in that** the mirror is composed of aluminum or an aluminum alloy.

22. The device according to any one of claims 19 to 21, **characterized in that** the diameter of the hollow cylinder is between 5 and 25 mm.

23. The device according to claim 22, **characterized in that** the diameter of the hollow cylinder is 20 mm.

24. The device according to any one of claims 19 to 23, **characterized in that** the laser is a diode laser that emits light of a wavelength of 750 to 1000 nm.

25. The device according to claim 24, **characterized in that** the laser emits light of a wavelength of 808 nm.

26. The device according to claim 24 or claim 25, **characterized in that** the laser has a power of 380 to 520 W.

27. The device according to claim 26, **characterized in that** the laser has a power of 500 W.

## Revendications

1. Procédé de soudage laser pour la connexion d'un segment de tuyau exempt de PVC à un corps moulé tubulaire en utilisant un laser et un miroir, dans lequel la paroi de tuyau du segment de tuyau est composée d'au moins deux couches de matériau différentes l'une de l'autre et une de ces couches contient au moins par section un absorbant pour rayonnement laser, dans lequel lors du procédé
a) le segment de tuyau exempt de PVC et le corps moulé tubulaire sont préparés,
b) le segment de tuyau et le corps moulé tubulaire sont insérés l'un dans l'autre par conjugaison de formes de sorte que les régions superficielles du segment de tuyau et du corps moulé tubulaire se chevauchent,
c) le segment de tuyau et le corps moulé tubulaire sont disposés devant le miroir avec les régions superficielles se chevauchant,
d) le segment de tuyau et le corps moulé tubulaire sont irradiés avec les régions superficielles se chevauchant avec un rayonnement laser de sorte que les rayons laser réfléchis ou transmis par le segment de tuyau ou par le corps moulé tubulaire sont réfléchis par le miroir et sont partiellement de nouveau incidents sur le segment de tuyau ou le corps moulé tubulaire, dans lequel une connexion soudée entre le segment de tuyau et le corps moulé tubulaire se forme.

2. Procédé selon la revendication 1, lors duquel le miroir a le contour de la face interne d'un cylindre creux.

3. Procédé selon une des revendications 1 ou 2, **caractérisé en ce que** l'absorbeur est contenu dans la couche formant la surface interne du segment de tuyau.

4. Procédé selon la revendication 3, **caractérisé en ce que** le corps moulé tubulaire est inséré dans le segment de tuyau à l'étape b).

5. Procédé selon une des revendications 1 ou 2, **caractérisé en ce que** l'absorbeur est contenu dans la couche formant la surface externe du segment de tuyau.

6. Procédé selon la revendication 5, **caractérisé en ce que** le segment de tuyau est inséré dans le corps moulé tubulaire à l'étape b).

7. Procédé selon une des revendications précédentes, **caractérisé en ce que** l'absorbeur est contenu à une concentration de 50 à 300 ppm dans la couche le contenant.

8. Procédé selon la revendication 7, **caractérisé en ce que** la couche contenant l'absorbeur se compose d'un matériau exempt de PVC.

9. Procédé selon la revendication 8, **caractérisé en ce que** le matériau est sélectionné parmi le polyéthylène, polypropylène, polyisoprène, des copolymères séquencés de styrène-oléfine comme SEBS, SEPS, SIS, SEB et des mélanges de ceux-ci.

10. Procédé selon une des revendications 7, 8 ou 9, **caractérisé en ce que** l'épaisseur de couche de la couche contenant l'absorbeur est de 20 à 100 µm.

11. Procédé selon une des revendications 1 à 6, **caractérisé en ce que** le matériau de la couche exempte d'absorbeur est sélectionné parmi le polyéthylène, polypropylène, polyisoprène, des copolymères séquencés de styrène-éthylène-butadiène-styrène (SEBS), des copolymères séquencés de styrène-éthylène-propylène-styrène (SEPS), des copolymères de styrène-isoprène-styrène (SIS), des copolymères de styrène-éthylène-butadiène (SEB) et des mélanges de ceux-ci.

12. Procédé selon une des revendications précédentes, **caractérisé en ce que** le segment de tuyau présente une épaisseur de paroi de 4 mm maximum.

13. Procédé selon une des revendications précédentes, **caractérisé en ce que** le corps moulé tubulaire est un connecteur ou un autre segment de tuyau.

14. Procédé selon la revendication 13, **caractérisé en ce que** le connecteur se compose essentiellement de polypropylène.

15. Procédé selon une des revendications précédentes, **caractérisé en ce que** le segment de tuyau comprend d'autres couches.

16. Procédé selon la revendication 15, **caractérisé en ce que** les autres couches sont perméables au rayonnement laser.

17. Connexion assemblée soudée au laser entre un segment de tuyau et un corps moulé tubulaire, fabriquée par le procédé selon une des revendications précédentes.

18. Système de tuyaux contenant une pluralité de connexions assemblées soudées au laser entre un segment de tuyau et un corps moulé tubulaire selon la revendication 17, destiné à être utilisé dans un circuit d'hémodialyse.

19. Dispositif de soudage de deux partenaires assemblés constitués d'un segment de tuyau et d'un corps moulé tubulaire qui comprend un laser et un miroir, dans lequel le laser, les partenaires assemblés et le miroir peuvent être disposés de sorte que le rayon du laser est incident sur les partenaires assemblés, puis sur le miroir, **caractérisé en ce que** le miroir présente partiellement le contour de la face interne d'un cylindre creux.

20. Dispositif selon la revendication 19, **caractérisé en ce que** le rayon du laser diverge dans une section de rayon et la section de rayon divergente est incidente sur les partenaires assemblés.

21. Dispositif selon la revendication 19 ou la revendication 20, **caractérisé en ce que** le miroir se compose d'aluminium ou d'un alliage d'aluminium.

22. Dispositif selon une des revendications 19 à 21, **caractérisé en ce que** le diamètre du cylindre creux se situe entre 5 et 25 mm.

23. Dispositif selon la revendication 22, **caractérisé en ce que** le diamètre du cylindre creux est de 20 mm.

24. Dispositif selon une des revendications 19 à 23, **caractérisé en ce que** le laser est un laser à diodes qui émet de la lumière d'une longueur d'onde de 750 à 1000 nm.

25. Dispositif selon la revendication 24, **caractérisé en ce que** le laser émet de la lumière d'une longueur d'onde de 808 nm.

26. Dispositif selon la revendication 24 ou la revendication 25, **caractérisé en ce que** le laser a une puissance de 380 à 520 W.

27. Dispositif selon la revendication 26, **caractérisé en ce que** le laser a une puissance de 500 W.
